(19) Europäisches Patentamt

European Patent Office

Office européen des brevets

(11) Publication number: **0 339 633**

**A2**

(12) **EUROPEAN PATENT APPLICATION**

(21) Application number: 89107623.4

(22) Date of filing: 27.04.89

(51) Int. Cl.4: **C07K 15/00 , C12P 21/00 , C12N 5/00 , C12N 15/00 , G01N 33/574 , G01N 33/577 , //(C12P21/00,C12R1:91)**

(30) Priority: 28.04.88 JP 105597/88

(43) Date of publication of application:
02.11.89 Bulletin 89/44

(84) Designated Contracting States:
DE FR GB

(71) Applicant: KYOWA HAKKO KOGYO CO., LTD.
6-1, Ohte-Machi 1-chome
Chiyoda-ku Tokyo-to(JP)

(72) Inventor: Yoshida, Hajime
9-18, Isobe
Sagamihara-shi Kanagawa(JP)
Inventor: Furuya, Akiko
I 15-405, 1210, Kisomachi
Machida-shi Tokyo(JP)

(74) Representative: Kinzebach, Werner, Dr. et al
Patentanwälte Reitstötter, Kinzebach und
Partner Sternwartstrasse 4 Postfach 86 06 49
D-8000 München 86(DE)

(54) Anti-human gastric cancer monoclonal antibody.

(57) An anti-human gastric cancer monoclonal antibody which belongs to the class IgM, reacts with human gastric cancer tissues but not with human normal tissues, recognizes unsialylated glycoproteins as antigens and shows no cross reactivity to carcinoembryonic antigen (CEA) and alpha-fetoprotein (AFP), a pathologic diagnosis of gastric cancer using said monoclonal antibody and a serodiagnosis or monitoring of gastric cancer, hepatic cancer, gallbladder carcinoma and/or biliary duct carcinoma using said monoclonal antibody.

EP 0 339 633 A2

# ANTI-HUMAN GASTRIC CANCER MONOCLONAL ANTIBODY

## BACKGROUND OF THE INVENTION

### FIELD OF THE INVENTION

This invention relates to a monoclonal antibody reactive with human gastric cancer and recognizing glycoproteins as antigens (epitopes). This monoclonal antibody is used in a method of making a pathologic diagnosis of gastric cancer and the antibody is also used in making a serologic diagnosis of or monitoring gastric cancer, hapatic cancer, gallbladder carcinoma and/or biliary duct carcinoma.

### BACKGROUND OF THE INVENTION

Serodiagnoses of digestive organ cancers using anti-human digestive organ cancer monoclonal antibodies have been reported [J. Clin. Immunol., 2, 135 (1982); Federation Proc., 41, 898 (1982); Cancer Res., 42, 601 (1982); Hybridoma, 2, 110 (1983); EP-A-235817 and EP-A-253646].

These monoclonal antibodies all recognize sialylated glycoproteins as antigens in the blood. In cancer serodiagnostic systems in which these monoclonal antibodies are used, the percentage of positive responses in pancreatic cancer cases is always higher than in other digestive organ cancer cases.

Alpha-fetoprotein (hereinafter abbreviated as AFP) is known to be a tumor marker in hepatic cancer and now is one of the most useful tumor markers for clinical diagnosis. While the percentage of positive responses in hepatic cancer obtained with AFP is 75-90%, AFP reportedly gives false positive results in 30-40% of hepatitis cases and in 30-50% of cirrhosis cases. In gastric cancer, AFP gives a positive response percentage in the order of several percent.

A monoclonal antibody reacting specifically with gastric cancer, if available, would be useful in gastric cancer diagnosis and usable the clinician to diagnose gastric cancer and correctly distinguish it from other conditions. While some monoclonal antibodies to gastric cancer are known, the advent of better antibodies is waited for.

### SUMMARY OF THE INVENTION

The present inventors found that a specified class of monoclonal antibodies produced by hybridomas derived, by cell fusion, from spleen cells of an antibody-producing mouse immunized with human gastric cancer tissue membrane components and cells of a mouse myeloma cell line has good reactivity to gastric cancer. Further, applicants describe a sandwich-technique enzyme immunoassay using this monoclonal antibody to be generally applicable in serodiagnoses of gastric cancer, hepatic cancer, gallbladder carcinoma and biliary duct carcinoma.

Thus the present invention provides an anti-human gastric cancer monoclonal antibody produced by preparing hybridomas by fusion of spleen cells of a mouse immunized with human gastric cancer tissue membrane components and cells of a mouse myeloma cell line, selecting hybridomas which can produce a monoclonal antibody having reactivity to the human gastric cancer cell membrane components, culturing these producer hybridomas in a medium or administering these hybridomas to mice to thereby culture the hybridomas in the ascitic fluid in mice and recovering the antibody from the culture or ascitic fluid.

### BRIEF DESCRIPTION OF THE DRAWING

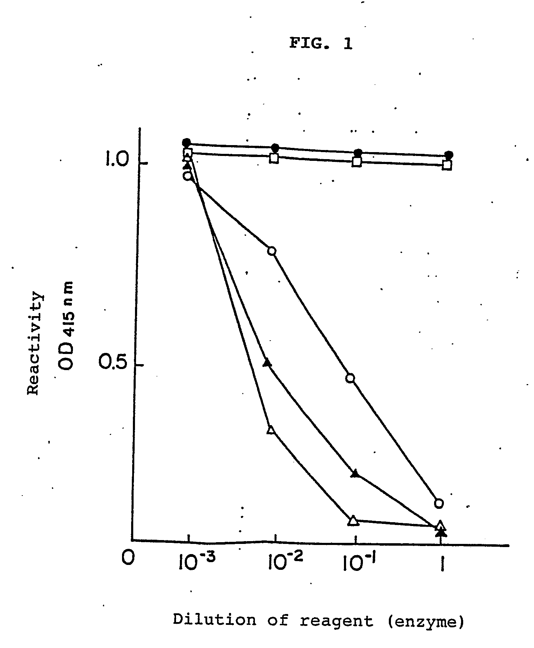

Fig. 1 shows the changes in reactivity of gastric cancer tissue-derived membrane components to the monoclonal antibody AMC-84 upon treatment with various enzymes and a reagent.

In the figure, the symbols used are as follows: ● for treatment with 0.1 U/ml neuraminidase, □ for treatment with 0.1 U/ml $\alpha$-L-fucosidase, o for treatment with 0.25% trypsin, ▲ for treatment with 10 U/ml protease, and △ for treatment with 50 mM $NaIO_4$.

## DETAILED DESCRIPTION OF THE INVENTION

The monoclonal antibody according to the invention belongs to the class IgM, reacts with gastric cancer cell membrane components and recognizes glycoproteins as antigens.

As a typical example of the monoclonal antibody according to the invention, there may be mentioned AMC-84, which is produced by the hybridoma cell line AMC-84 (deposited at the Fermentation Research Institute, Agency of Industrial Science and Technology as of February 25, 1988 under the deposit number FERM BP-1761 in accordance with the Budapest Treaty).

In the following, a process for producing the monoclonal antibody according to the invention is described in detail.

### (1) Immunization of animal and preparation of antibody-producing cells

Mice 3-10 weeks of age, preferably 8-week-old mice, are immunized with human gastric cancer cells, tissues or membrane components to prepare antibody-producing cells in the spleen, lymph nodes and peripheral blood of the animals. Mice that have immunological tolerance as a result of pretreatment with normal human stomach cells should preferably be used as the mice to be immunized. The immunization is performed generally by administering human gastric cancer cells ($10^6$ to $10^7$ cells per animal), human gastric cancer tissues, or membrane components (membrane fragments) derived therefrom (10-500 $\mu$g per animal) together with an appropriate adjuvant (e.g. complete Freund's adjuvant, or aluminum hydroxide gel plus pertussis vaccine) to the animals subcutaneously, intravenously or intraperitoneally. Thereafter, antigen administration is repeated 2-5 times at 1- to 2-week intervals. Three to seven days after each immunization, the blood is sampled from the eyeground venous plexus and the serum of each sample is tested as to whether it reacts with human gastric cancer by the enzyme immunoassay technique described below [Enzyme-linked Immunosorbent Assay (ELISA), published by Igaku Shoin, Tokyo, 1976], for instance.

Enzyme immunoassay technique:

The membrane components of normal or tumor cells or tissues (membrane fragment fraction containing 10-1,000 $\mu$g of proteins per milliliter) are distributed into wells of a 96-well plate for EIA (Flow Laboratories) (100-200 $\mu$l per well). After allowing to stand at 4°C for 15 to 40 hours, each well is deprived of the supernatant and, then, washed well with deionized water or phosphate-buffered saline (PBS; 1.83 g of disodium phosphate, 0.21 g of monopotassium phosphate and 7.65 g of sodium chloride in 1 liter of distilled water, pH 7.2). Then, 1% BSA (bovine serum albumin)-PBS is distributed into the wells (100-200 $\mu$l per well) and protein binding residues remaining on the plate are blocked by allowing to stand at 4°C for 15 to 40 hours. After discarding the BSA-PBS, the wells are washed well with deionized water or PBS. Samples (mouse sera, hybridoma culture supernatants, or purified monoclonal antibodies; each as the first antibody) are diluted with BSA-PBS and the dilutions are distributed into the wells (100 $\mu$l per well), followed by standing at 4°C for 15 hours. After washing the wells with one portion of deionized water and then with 6 portions of 2 M NaCl solution, a 100-fold dilution of a rabbit anti-mouse immunoglobulin IgG-peroxidase conjugate (produced by DAKO and distributed by Kyowa Medex; used as the second antibody) is distributed into the wells (100 $\mu$l per well). The plate is then allowed to stand at room temperature for 2 hours.

After washing the wells thoroughly with PBS, an ABTS substrate solution [prepared by dissolving 550 mg of 2,2'-azinobis(3-ethylbenzothiazoline-6-sulfonic acid) diammonium salt in 1 liter of 0.1 M citrate buffer (pH 4.2) and adding, just prior to use, hydrogen peroxide to a concentration of 1 $\mu$l/ml] is applied and the color developed is measured in terms of the absorbance $OD_{415nm}$. Those mice that strongly react with the

3

gastric cancer cells, tissues or membrane components are used as human gastric cancer-immunized mice, namely as sources of supply of antibody-producing cells for hybridoma production.

When cells per se are used as the antigen in performing enzyme immunoassay, the target cells are cultured on a Falcon 3072 plate, 0.25% glutaraldehyde-PBS is added and, after allowing it to stand at room temperature for 1-2 hours, the plate is washed well with PBS. Then, 100-200 $\mu$l of 1% BSA-PBS is added and, after 2 hours of standing, the plate is washed well with deionized water or PBS and submitted to antibody titer determination, which is conducted in the same manner as the case where an ordinary antigen-coated plate is used.

For submitting to cell fusion, human gastric cancer cells, tissues or membrane components are intraperitoneally administered to mice to be immunized in a dose of 2 to 5 x 10$^6$ cells per animal or 20 to 400 $\mu$g per animal 3-4 days prior to the fusion treatment. The spleen is excised, cut into fragments in Eagle's minimal essential medium (MEM; Nissui Pharmaceutical), loosened up with a pair of forceps, and centrifuged at 1,200 rpm for 5 minutes. The supernatant is discarded, and the cells obtained as the sediment are deprived of erythrocytes by 1- to 2-minute treatment with Tris-ammonium chloride buffer (pH 7.65), washed with three portions of MEM, and used as the spleen cells for fusion.

The human gastric cancer tissue membrane components preparation is performed in the following manner.

An appropriate tissue piece stored frozen at -80°C is thawed, then cut to pieces with scissors, disrupted mechanically in an ultra-Disperser (manufactured by Yamato), homogenized with a glass-Teflon homogenizer, and centrifuged at 4°C and 100,000 x g for 20 minutes. The supernatant is further centrifuged at 4°C and 100,000 x g for 1 hour. The sediment thus obtained is suspended in 0.01 M phosphate buffer (pH 7.2) for use as the membrane components.

(2) Preparation of myeloma cells

A mouse-derived established myeloma cell line is used. Suitable examples of such cell lines are the 8-azaguanine resistant (BALB/c-derived) mouse myeloma cell lines P3-X63Ag8-U1 (P3-U1) [Current Topics in Microbiology and Immunology-1] [European J. Immunology, 6, 511-519 (1976)], SP2/0-Ag14 (SP-2) [Nature, 276, 269-270 (1978)], P3-X63-Ag8653 (653) [J. Immunology, 123, 1548-1550 (1979)] and P3-X63-Ag8 (X63) [Nature, 256, 495-497 (1975)]. These cell lines are subcultured in 8-azaguanine medium (normal medium prepared by adding, to RPMI-1640 medium, glutamine (1.5 mM), 2-mercaptoethanol (5 x 10$^{-5}$ M), gentamicin (10 $\mu$g/ml) and fetal calf serum (FCS; produced by CSL) (10%), with further supplementation with 8-azaguanine (15 $\mu$g/ml)]. The cell line selected for cell fusion should be transferred to normal mediun 3-4 days before cell fusion to ensure the cell count of not less than 2 x 10$^7$ on the day of fusion.

(3) Cell fusion

The antibody-producing cells immunized in (1) and the myeloma cells obtained in (2) are washed well with MEM or PBS and mixed in a cell number ratio of antibody-producing cells:myeloma cells = 5 to 10:1 and then subjected to centrifugation (1,200 rpm, 5 minutes). The supernatant is discarded and the cell sediment is loosened up. With stirring at 37°C, a mixture of 2 g of polyethylene glycol 1000 (PEG-1000), 2 ml of MEM and 0.7 ml of dimethyl sulfoxide is added thereto in an amount of 0.2-1 ml per 10$^3$ antibody-producing cells and, following several additions of 1-2 ml of MEM at 1-to 2-minute intervals, the whole volume is made 50 ml by adding MEM. After centrifugation (900 rpm, 5 minutes), the supernatant is discarded and the cell sediment is loosened gently. To the cells is added 100 ml of normal medium (RPMI 1640 with 10% FCS). The cells are suspended in the medium by gentle drawing up into and discharging from a measuring pipette.

The suspension obtained is distributed, in 100 $\mu$l-portions, into each well of a 96-well incubation plate. Incubation is carried out in a 5% CO$_2$ incubator at 37°C for 24 hours. HAT medium (normal medium supplemented with hypoxanthine (10$^{-4}$ M), thymidine (1.5 x 10$^{-5}$ M) and aminopterine (4 x 10$^{-7}$ M)] is added to the incubation plate (100 $\mu$l per well) and incubation is conducted for another 24 hours. Thereafter, the culture supernatant (100 $\mu$l) is discarded and the same volume of fresh HAT medium is added instead at 24-hour intervals for 2 days. The incubation in the CO$_2$ incubator at 37°C is continued for 10-14 days.

For those wells in which fused cells grown and forming colonies are found, the supernatant (100 $\mu$l) is discarded and the same volume of HT medium (HAT medium minus aminopterine) is added, followed by medium replacement with fresh portions of HT medium at 24-hour intervals for 2 days.

4

After 3-4 days of cultivation in HT medium, a portion of the culture supernatant is collected and assayed for antibody titer relative to human gastric cancer by the above-mentioned enzyme immunoassay technique. Simultaneously, the reactivities to normal human cells or tissues or membrane components thereof, among others, are also determined in the same manner, and those wells for which specific reactivity is found to human gastric cancer cells or tissues or membrane components thereof are selected. For the wells showing strong reactivity to human gastric cancer cells or tissues or membrane components thereof but no reactivity to normal human cells or tissues or membrane components thereof, among others, cloning is repeated twice by the limiting dilution technique. In this way, those clones for which high antibody titer values are stably obtainable relative to human gastric cancer cells or tissues or membrane components thereof are selected as anti-human gastric cancer monoclonal antibody-producing hybridoma cell lines.

(4) Preparation of monoclonal antibody

Eight- to ten-week-old female nude mice treated with pristane [intraperitoneally administered with 0.5 ml of 2,6,10,14-tetramethylpentadecane (pristane) and fed for 2-weeks] are intraperitoneally injected with the anti-human gastric cancer monoclonal antibody-producing hybridomas obtained in (3) at a dose of $2-4 \times 10^6$ cells per animal. In 10-21 days, the hybridomas cause ascites tumor in the mice. The ascitic fluid is collected from such a mouse, centrifuged (3,000 rpm, 5 minutes) to remove solids, subjected to salting out with 50% ammonium sulfate, dialyzed against PBS supplemented with 0.5 M NaCl, and applied to a Cellulofine GSL-2000 (Seikagaku Kogyo) colum (bed volume 750 ml) at a flow rate of 15 ml/hr. An IgM fraction is collected and used as a purified monoclonal antibody.

The isotype of the antibody is determined by Ouchterlony's method (double immunodiffusion) [Seibutsukagaku Jikkenho (Methods in Experimental Biochemistry), vol. 15, Introduction to Experimental Immunology, p. 74, Gakkai Shuppan Center, 1981].

The protein quantity is estimated by the Folin method, followed by calculation based on the absorbance at 280 nm [1.0 ($OD_{280}$) is approximately equal to 1 mg of immunoglobulin per milliliter].

The monoclonal antibodies thus obtained are evaluated for specificity characaeristics based on the reactivities to normal and tumor tissues and membrane components thereof derived from a variety of human organs obtained from a plurality of subjects, the reactivities to a variety of cultured human normal or tumor cell lines or cultured human fetal cell lines or membrane components derived therefrom, and the reactivity to the hitherto known carcinoembryonic antigen (CEA) as determined by the enzyme immunoassay method, fluorescent antibody method, immunohistological evaluation method (ABC method), etc.

(5) Serodiagnosis

The serodiagnosis is performed as follows:

A first antibody preparation (10-100 μg/ml) is distributed into the wells of a 96-well plate for EIA (50-200 μl per well). The plate is allowed to stand at 4°C for 15 to 40 hours or at room temperature for 2-4 hours. After washing with PBS, 200 μl of BSA-PBS is added to each well, followed by further standing at 4°C for 15 hours or at room temperature for 2 hours. The plate is then washed well with PBS, and 50-100 μl of a 1- to 100-fold dilution of a serum sample is added to each well. After allowing it to stand at 4°C for 15 hours or at room temperature for 2 hours, the plate is washed well with PBS. Then, a biotinylated or peroxidase-labeled second antibody (10-100 μg/μl) is added to the wells (50-100 μl per well) and the plate is further allowed to stand at 4°C for 15 hours or at room temperature for 2-4 hours. When a biotinylated antibody is used as the second antibody, the plate is washed well with PBS, avidin-peroxidase or avidin-biotin-peroxidase (10 μg/ml) is added to the wells (50-100 μl per well), and the plate is allowed to stand at room temperature for 30 minutes and then washed well with PBS. Then, an ABTS substrate solution is added, as the substrate solution, in an amount of 50-100 μl per well. After allowing the plate to stand at room temperature for 10-30 minutes, the reaction is terminated by adding 5% SDS solution in an amount of 50-100 μl per well. The $OD_{415}$ value is measured for each well and the antigen quantity in the serum sample is calculated based on the intensity of the color developed. By comparing the antigen levels in the sera of healthy humans with those in the sera of patients with various kinds of cancer, a normal level range is defined. When the level in question exceeds such range, the test is regarded as positive.

The serodiagnostic method described above can be used also in evaluating the effects of cancer therapy employed or predicting the recurrence of cancer by measuring at appropriate time intervals (i.e. monitoring) the serum cancer antigen level in a patient.

(6) Antigen analysis

When in performing the above-mentioned enzyme-linked antibody method, or immunohistochemical staining, the antigens (gastric cancer membrane components, cultured gastric cancer cell lines, gastric cancer tissues) are retreated with reagents such as enzymes (e.g. neuramini dase, protease) or periodic acid and then reacted with the monoclonal antibodies, the subsequent comparison for differences in reactivity to the monoclonal antibodies between the original antigens without such pretreatment and the antigens pretreated in the above manner can shed light on the chemical characteristics of the antigens, that is the chemical characteristics of the antigenic sites which the monoclonal antibodies recognize. Thus, if the antigenicity disappears upon treatment with neuraminidase, it is estimated that sialic acids are associated with the antigenic determinants. If the antigenicity disappears upon protease treatment, the involvement of proteins in the antigenic determinants is probable. If the antigenicity disappears upon periodic acid treatment, sugar chains are presumably associated with the antigenic determinants.

The pathologic diagnosis using the monoclonal antibody according to the invention is performed by the immunohistochemical staining method [the ABC method described in "Koso-Kotai-Ho (Enzyme-Antibody Method)" (edited by K. Watanabe and K. Nakane, published by Gakusai Kikaku, 1985), page 106].

The following examples are presented to further illustrate the invention in further detail, but are not intended to be construed to limit the scope of the invention.

## EXAMPLE 1

(1) Preparation of antibody-producing cells

Normal human stomach membrane components were administered intravenously to new-born BALB/c mice (Experimental Animal Agricultural Cooperative Association of Shizuoka prefecture) within 24 postnatal hours at a dose of 1 mg of proteins per animal. After the lapse of 8 weeks, the mice were intraperitoneally administered with human gastric cancer membrane fragments (100 μg as proteins per animal) together with aluminum hydroxide gel (2 mg per animal) and killed pertussis vaccine ($1 \times 10^9$ per animal), followed by 3-5 immunizations with the same antigen at a dose of 100 μg per animal on the protein basis at 1- to 2-week intervals. From among these immunized mice, those mice whose antisera intensely reacted with human gastric cancer cells or tissues or membrane fragments derived therefrom were selected as the desired immunized mice, and spleen cells were prepared from such mice and submitted to cell fusion.

(2) Preparation of mouse myeloma cells

The 8-azaguanine-resistant mouse myeloma cell line P3-U1 was cultivated in normal medium to thereby secure not less than $2 \times 10^7$ cells at the time of cell fusion, and submitted to cell fusion as a parent strain.

(3) Hybridoma production

The spleen cells and myeloma cells obtained in (1) and (2), respectively, were used in a ratio of 5:1 and subjected to fusion following the procedure mentioned hereinabove. After cultivation in HAT medium at 37° C under 5% $CO_2$ for 14 days, fused cells were selected and, after change of the medium to HT medium, cultivation was continued. Based on the results of anti-human gastric cancer antibody titer determination, active wells were selected and, after change of the medium to normal medium, cloning was repeated twice. Thus was selected the hybridoma cell line AMC-84 capable of producing a monoclonal antibody having no reactivity to any of normal human cells or tissues but having specific reactivity to human gastric cancer, as determined by enzyme immunoassay or immunohistological evaluation ABC method).

(4) Monoclonal antibody purification

Pristane-treated 8-week-old female nude mice were intraperitoneally injected with the hybridoma cell line AMC-84 obtained in (3) at a dose of 4 x 10⁶ cells per animal. In 10-21 days, the hybridoma caused ascites tumors. The ascitic fluid was collected from ascitic fluid-bearing mice (5-10 ml per animal), deprived of solids by centrifugation (3,000 rpm, 5 minutes), subjected to salting out with 50% ammonium sulfate, dialyzed against PBS supplemented with NaCl (0.5 M), and applied to a Cellulofine GSL2000 column (bed volume 750 ml) at a flow rate of 15 ml/hr. An IgM fraction was collected and used as a purified monoclonal antibody.

(5) Specificity of AMC-84

The results of testing of the thus-obtained anti-human gastric cancer monoclonal antibody AMC-84 for reaction specificity are summarized below in Table 1.

The measurements were performed by the ELISA method as follows:

When tissues (membrane components) were used as targets:

A tissue (membrane components) suspension was distributed into the wells of a 96-well plate for enzyme immunoassay (manufactured by Linbro) (50 μl per well), and the plate was allowed to stand at 37°C for 2 hours or at 4°C for 15 hours for tissue (membrane components) fixation. After washing the plate with PBS, PBS containing 10% FCS was distributed into the wells (100 μl per well) for protecting active residues of the fixed tissue (membrane components). After washing the plate with PBS, the first antibody AMC-84 was distributed into the wells (50 μl per well) and the plate was allowed to stand at 37°C for 1-2 hours or at 4°C for 15 hours to allow the target and antibody to react with each other. The antibody portion remaining unreacted was removed by five washings with PBS containing 0.05% Tween-20 (Wako Pure Chemical Industries). Peroxidase-bound rabbit anti-mouse immunoglobulin (Miles-Yeda; 200-fold dilution) (used as the second antibody) was distributed into the wells (50 μl per well) and the reaction was allowed to proceed at 37°C for 1 hour. The plate was then washed 5 times with PBS containing 0.05% Tween-20 and 3 times with deionized water.

An ABTS substrate solution was added to the wells (50 μl per well) for initiating the reaction and, for terminating the reaction, 5% aqueous sodium lauryl sulfate solution was added (50 μl per well).

When cultured cell lines were used as targets:

Cells were cultured on a 96-well culture plate (Linbro) and, when they were confluent, they were reacted with the first and second antibodies in the same manner as mentioned above for tissues (membrane components). The conditions with the first and second antibodies were each conducted at room temperature for 30 minutes. After color development, the reaction was terminated by transferring each liquid reaction mixture to a well of a 96-well plate for analysis.

When the antigens CEA and AFP were used, the tests were performed in the same manner as in the tissue cases by using CEA and AFP, respectively, in lieu of the tissue.

In each case, the absorbance at 415 nm was measured with the wavelength 490 nm as the reference wavelength.

7

## Table 1

| | Sample | Reactivity[a] |
|---|---|---|
| Tissue (membrane components) | Gastric cancer | 26/32 |
| | Normal stomach | 0/ 2 |
| | Other normal tissues [b] | 0/11 |
| Cultured cell line | Lung cancer | 1/8 |
| | Pancreatic cancer | 0/1 |
| | Colonic cancer | 2/4 |
| | Gastric cancer | 3/6 |
| | Other cancers[c] | 0/5 |
| Antigen | C E A | 0/1 |
| | A F P | 0/1 |

a) Number of positive cases/number of samples
b) Liver, kidney, stomach, pancreas, intestine, spleen, heart, bone marrow, lymphocyte, erythrocyte, brain
c) Uterine cancer, prostatic cancer, melanoma.

As shown in Table 1, AMC-84 has high reactivity to gastric cancer. It is seen, however, that since it reacts neither with CEA nor with AFP, it differs from anti-CEA antibodies and from anti-AFP antibodies.

### EXAMPLE 2

Formalin-fixed, paraffin-embedded tissue sections (sliced to a thickness of 5 $\mu$m with a microtome) of various normal adult and fetal organs and cancer tissues were each fixed on an egg albumin-coated slide glass, deparaffinized with xylene and rendered hydrophilic stepwise with alcohol-water. Each section was rinsed with deionized water for 5 minutes and then allowed to stand in methanol containing 0.3% $H_2O_2$ at room temperature for 30 minutes for blocking endogenous peroxidases. The section was then washed with PBS for 20 minutes and allowed to stand in diluted normal mouse serum at room temperature for 20 minutes. The excess serum was sucked off from the section, and the section was reacted with the first antibody (anti-human gastric cancer monoclonal antibody AMC-84, 10 $\mu$g/ml) for 30 minutes. After washing with PBS, the section was then reacted with a diluted biotinylated antibody (biotinylated rabbit anti-IgG antibody, product of Vector) for 30 minutes, washed further with PBS, and reacted with an avidin-biotin-peroxidase complex (Vector) for 30 minutes. After washing well with PBS, the section was reacted with a peroxidase substrate [0.1% diaminobenzidine tetrahydrochloride prepared by using 0.1 M Tris-hydrochloride buffer (pH 7.2) containing 0.02% $H_2O_2$] for 2 minutes. The reaction was then terminated by cooling in ice. The section was stained with hematoxylin, dehydrated with alcohol-water and xylene, fixed with Canadian balsam, and observed under a microscope. The results thus obtained are shown in Table 2.

## Table 2

| Tissue | Positive cases/samples | Percent positiveness |
|---|---|---|
| Scirrhous gastric carcinoma | 1/1 | 100[a] |
| Gastric signet ring cell carcinoma | 1/1 | 100[a] |
| Gastric adenocarcinoma | 8/8 | 100[a] |
| Colonic cancer | 3/5 | 60[b] |

a) The cell membrane and cytoplasm of the cancer cell were stained. In the case of adenocarcinoma, the contents of the adenic cavity were stained.
b) The cancer cell and adenic cavity contents were partly stained.

As shown in Table 2, AMC-84 strongly reacted with gastric cancer in high percentage, although it weakly reacted with some colonic cancer cells. With normal fetal tissues (heart, pancreas, stomach, large intestine, kidney, thyroid gland, brain, spleen, liver, small intestine, etc.), it reacted very weakly with some cells. It did not react with any other tissues at all.

## EXAMPLE 3

For analyzing the antigens which the monoclonal antibody AMC-84 recognizes, gastric cancer tissue-derived membrane components were treated with the enzymes and reagent described below and then examined for the reactivity with AMC-84.

Enzymes and reagent

Trypsin (2.5% solution; Gibco)
0.25% in PBS
Neuraminidase (Boehringer Mannheim)
0.1 U/ml in 0.1 M acetate buffer (pH 4.5)-3 mM $CaCl_2$
α-L-Fucosidase (Boehringer Mannheim)
0.1 U/ml in 0.1 M phosphate buffer (pH 6.3)
Protease (Sigma)
10 U/ml in 0.1 M phosphate buffer (pH 7.2)
$NaIO_4$ (Wako Pure Chemical Industries)
50 mM in PBS

Thus, the gastric cancer tissue membrane components (10 μg of proteins per ml) were distributed, in 50-μl portions, into each well of a plate for EIA (Linbro). After standing at 4°C for 15 hours, the plate was washed three times with PBS. Then, 1% BSA-PBS was distributed into the wells (100 μl per well). The plate was allowed to stand at room temperature for 30 minutes to 2 hours and then washed three times with PBS. One of the above enzyme solutions or reagent solution was distributed into the wells (50 μl per well) and the reaction was carried out at 37°C for 1 hour. Then, the plate was washed five times with PBS and the monoclonal antibody AMC-84 (10 μg/ml) was distributed into the wells (50 μl per well), followed by allowing to it stand at 4°C for 15 hours.

After washing five times with Tween 20-PBS (Tween 20 being a product of Wako Pure Chemical Industries), peroxidase-labeled anti-mouse IgG (product of DAKO, distributed by Kyowa-Medix; 400-fold dilution) was added (50 μl per well), and the reaction was carried out at room temperature for 2 hours. The plate was washed five times with Tween 20-PBS, the ABTS substrate solution was added (100 μl), the reaction was conducted for 30 minutes, and the absorbance was measured at 415 nm.

As shown in Fig. 1, the antigenicity completely disappeared upon treatment with $NaIO_4$, trypsin or protease. Based on these results, it was estimated that the monoclonal antibody AMC-84 recognizes unsialylated glycoproteins.

## EXAMPLE 4

A suspension of AMC-84 (10 μg/ml) was distributed in 50-μl portions into each well of a 96-well plate for EIA (Flow Laboratories). After standing at 4°C for 15 hours, the plate was washed with PBS. Then, 1% BSA-PBS was added (200 μl per well). After standing for 15 hours, the plate was washed well with PBS. To the plate were added 10-fold dilutions of normal human-derived sera (144 samples) or of gastric cancer patient-derived sera (140 samples) in an amount of 50 μl per well. After standing at 4°C for 15 hours, the plate was washed well with PBS. Then, biotinylated anti-gastric cancer monoclonal antibody AMC-84 (10 μg/ml) was added as the second antibody (100 μl per well). The plate was allowed to stand at 4°C for 15 hours and, then, washed well with PBS. Avidin-peroxidase (product of Vector) (10 μg/ml) was distributed in 100-μl portions into each well , and the plate was allowed to stand at room temperature for 1 hour and then washed with PBS. Thereafter, the ABTS substrate solution was added in an amount of 100 μl per well and the reaction was allowed to proceed at room temperature for 30 minutes and then terminated by adding 5% SDS (sodium dodecyl sulfate) solution (100 $\mu l$ per well). For each well, the color development was determined by measuring the absorbance at 415 nm ($OD_{415}$). As shown in Table 3, positive results ($OD_{415}$ > 0.05) were obtained only for 3 (2%) out of the 144 serum samples from healthy subjects, without any positive results in the remaining 141 cases.

On the other hand, 13 (27%) out of 78 samples from gastric cancer patients gave positive results. In hepatic cancer patients, 9 (75%) out of 12 samples gave positive results and, in gallbladder and/or biliary duct carcinoma patients, 10 (52.6%) out of 19 samples reacted positive. In pancreatic cancer patients, only 2 (22%) out of 9 samples gave positive results.

Table 3

| Serum sample | Positive results*/samples | Percent positiveness |
|---|---|---|
| Healthy subjects | 3/144 | 2 |
| Gastric cancer | 21/78 | 26.9 |
| Pancreatic cancer | 2/9 | 22.0 |
| Hepatic cancer | 9/12 | 75 |
| Colonic cancer | 0/10 | 0 |
| Rectal cancer | 1/10 | 10 |
| Gallbladder/biliary duct carcinoma | 10/19 | 52.6 |
| Breast cancer | 0/2 | 0 |

* When the $OD_{415}$ value was not less than 0.05, the result was judged as positive.

The above results indicate that the antibody according to the invention is effective in making a serodiagnosis of gastric cancer, gallbladder/biliary duct carcinoma and, in particular, hepatic cancer.

The invention thus provides a monoclonal antibody effective in making a pathologic diagnosis of gastric cancer or a serodiagnosis of gastric cancer, gallbladder and/or biliary duct carcinoma and, in particular, hepatic cancer.

While the invention has been described in detail and with reference to specific embodiments thereof, it will be apparent to one skilled in the art that various changes and modifications can be made therein wituout departing from the spirit and scope thereof.

## Claims

1. An anti-human gastric cancer monoclonal antibody which belongs to the class IgM, reacts with human gastric cancer tissues but not with normal human tissues, recognizes unsialylated glycoproteins as antigens and shows no cross reactivity to carcinoembryonic antigen (CEA) and alpha-fetoprotein (AFP).

2. An anti-human gastric cancer monoclonal antibody secreted by a hybridoma having the characteristics of FERM BP-1761 and reactive with human gastric cancer tissues but not with normal human tissues.

3. A method for detecting the presence of gastric cancer in a patient-derived tissue sample comprising:

(a) subjecting a sample of the patient's stomach tissue or membrane components thereof to enzyme immunoassay with the monoclonal antibody which belongs to the IgM class, reacts with human gastric cancer tissues but not with normal human tissues, recognized unsialyated glycoproteins as antigens and shows no cross reactivity to carcinoembryonic antigen and alpha-fetoprotein; and

(b) examining the results for the presence of a positive reaction.

4. A method of detecting the presence of gastric cancer, hepatic cancer, gallbladder carcinoma or biliary duct carcinoma in a patient's serum sample comprising:

(a) subjecting a serum sample from a patient to enzyme immunoassay with a monoclonal antibody which belongs to the IgM class, reacts with human gastric cancer tissues but not with normal human tissues, recognized unsialyated glycoproteins as antigens and shows no cross reactivity to carcinoembryonic antigen and alpha-fetoprotein; and

(b) examining the results for the presence of a positive reaction.

FIG. 1

Dilution of reagent (enzyme)